# EUROPEAN PATENT APPLICATION

(11) **EP 4 362 038 A1**
(43) Date of publication of application: **01.05.2024**
(21) Application number: 22828572.2
(22) Date of filing: 04.04.2022
(51) Int. Cl.: G16H 20/17, G16H 50/20, G16H 10/60

(54) **METHOD, DEVICE, AND COMPUTER PROGRAM PRODUCT FOR CALCULATING OPTIMAL INSULIN INJECTION AMOUNT**

(30) Priority: 21.06.2021 KR 20210079758
(71) Applicant: Eoflow Co., Ltd., Seongnam-si, Gyeonggi-do 13605 (KR)
(72) Inventor: JEON, Ho Min, Yongin-si Gyeonggi-do 16838 (KR); HAN, Sangjin, Gunpo-si Gyeonggi-do 15870 (KR)
(74) Representative: TBK
(86) International application number: PCT/KR2022/004773
(87) International publication number: WO 2022/270720

(57) **Abstract**

A method, device, and computer program product for calculating an optimal insulin injection amount is provided, wherein a glucose measurement value for a first user is received from a biometric value measurement device, information about food eaten is received from the first user, and an insulin injection amount is calculated on the basis of the glucose measurement value and the information about food.

## Description

### [Technical Field]

The present disclosure provides a method, device, and computer program product for calculating an optimal insulin injection amount.

### [Background Art]

Diabetes mellitus is a metabolic disorder that causes symptoms in which glucose levels are out of a normal range due to an insufficient secretion of insulin or a malfunction in a normal function thereof. Diabetes is a complex disease that may affect each tissue of the human body due to complications such as blindness, renal failure, heart failure, and neuropathy, and the number of diabetic patients is reported to be increasing every year.

In the case of diabetes, glucose needs to be measured by using glucose measurement devices, glucose needs to be managed through appropriate means such as diet, exercise program, insulin injection, and oral diabetes medicine.

Recently, a technology for automatically injecting insulin into diabetic patients by using drug injection devices has been studied, and there is a need for a technology capable of calculating an optimal insulin injection amount or insulin injection time point to be injected from drug injection devices to prevent over-injection of insulin, or the like.

### [Detailed Description of Disclosure]

### [Technical Problem]

The present disclosure provides a method, device, and computer program product for calculating an optimal insulin injection amount. The technical problems to be achieved by the present embodiment are not limited to the technical problems described above, and other technical problems may be inferred from the following embodiments.

### [Technical Solution]

According to an aspect of the present disclosure, a method of calculating an insulin injection amount may include: receiving a glucose measurement value for a first user from a biometric value measurement device; receiving, from the first user, information about food eaten; and calculating an insulin injection amount on the basis of the glucose measurement value and the information about food.

Also, the information about food may include at least one of information about the type of food, information about a restaurant selling the food, and information about a point in time of eating the food.

In addition, the calculating may include: determining whether or not the food is the same as food eaten by the first user in the past; and calculating the insulin injection amount on the basis of the determination, wherein, when the food is determined to be the same as the food eaten by the first user in the past, the insulin injection amount is calculated on the basis of at least one of an insulin injection amount injected into the first user after the first user eats the food in the past and information about a change in glucose measurement value for the first user after the first user eats the food in the past.

Also, the calculating may include: determining whether or not the food is the same as food eaten by the first user in the past; and calculating the insulin injection amount on the basis of the determination, wherein, when the food is determined not to be the same as the food eaten by the first user in the past, the insulin injection amount is calculated on the basis of an insulin injection amount injected into a second user after the second user eats the food in the past, information about a change in glucose measurement value for the second user after the second user eats the food in the past, and a carbohydrate-to-insulin ratio set for each user.

In addition, the calculating may include: calculating an error range on the basis of a difference value between a glucose measurement value received from the biometric value measurement device and a glucose measurement value measured by a blood glucose monitoring (BGM) method; and calculating an insulin injection amount on the basis of the error range.

Another aspect of the present disclosure may provide a device for calculating an insulin injection amount including: a memory configured to store at least one program; and a processor configured to perform a calculation by executing the at least one program, wherein the processor is configured to receive a glucose measurement value for a first user from a biometric value measurement device, receive, from the first user, information about food eaten, and calculate an insulin injection amount on the basis of the glucose measurement value and the information about food.

Another aspect of the present disclosure may provide a computer program product including one or more computer-readable recording media configured to store a program for performing: receiving a glucose measurement value for a first user from a biometric value measurement device; receiving, from the first user, information about food eaten; and calculating an insulin injection amount on the basis of the glucose measurement value and the information about food.

### [Advantageous Effects of Disclosure]

According to one of the problem-solving means of the present disclosure described above, an insulin injection amount calculated by a user roughly inputting an amount of carbohydrates according to a type of food may not be accurate, and thus, a method capable of calculating an optimal insulin injection amount by receiving information about food eaten by a user may be provided.

Also, according to one of the problem-solving means of the present disclosure, even when food eaten by a first user corresponds to food eaten for the first time, a method capable of calculating an optimal insulin injection amount by using information related to a second user other than the first user may be provided.

In addition, according to one of the problem-solving means of the present disclosure, a glucose measurement value acquired from a glucose measurement device may have an error range according to an error of the glucose measurement device or physical characteristics of a user, and thus, a method capable of calculating an optimal insulin injection amount by considering an error range may be provided.

### [Brief Description of Drawings]

FIG. 1 is a block diagram of an insulin management system including a user terminal, a controller, and a drug injection device.
FIG. 2 is a view illustrating an internal structure of a drug injection device according to an embodiment.
FIGS. 3A and 3B are example views illustrating a method of setting up a drug injection amount calculator, according to an embodiment.
FIG. 4 is an example view illustrating calculating an insulin injection amount on the basis of information about food, according to an embodiment.
FIG. 5 is an example view illustrating calculating an insulin injection amount on the basis of information about food and information about a second user, according to an embodiment.
FIG. 6 is an example view illustrating calculating an insulin injection amount on the basis of an error range, according to an embodiment.
FIG. 7 is a flowchart of a method of calculating an insulin injection amount, according to an embodiment.

### [Best Mode]

A method, device, and computer program product for calculating an optimal insulin injection amount may be provided.

In the present disclosure, a glucose measurement value for a first user may be received from a biometric value measurement device, information about food eaten may be received from the first user, and an insulin injection amount may be calculated on the basis of the glucose measurement value and the information about food.

### [Mode for Disclosure]

Hereinafter, embodiments of the present disclosure will be described in detail with reference to the accompanying drawings, so that those skilled in the art may easily practice the present disclosure. However, the present disclosure may be embodied in many different forms and is not limited to the embodiments described herein. Also, in order to clearly describe the present disclosure in the drawings, elements irrelevant to the description are omitted, and like reference numerals denote like elements throughout.

As used herein, when an element is referred to as being "connected to" or "coupled to" another element, it may be directly connected or coupled to the other element or may be electrically connected to the other element with intervening elements. In addition, when a certain element is referred to as "comprising" or "including" another element, this means that it may further include other elements rather than excluding the other elements unless specifically stated otherwise.

Hereinafter, the present disclosure will be described in detail with reference to the accompanying drawings.

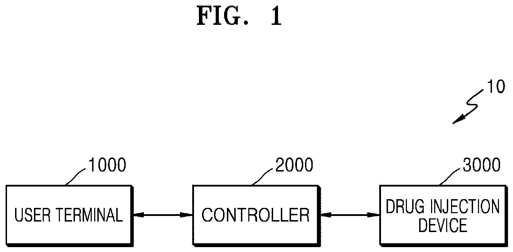

FIG. 1 is a block diagram of an insulin management system including a user terminal, a controller, and a drug injection device.

An insulin management system 10 according to an embodiment may include a user terminal 1000, a controller 2000, and a drug injection device 3000. Meanwhile, FIG. 1 illustrates that the insulin management system 10 includes only components related to an embodiment. Therefore, it is apparent to those skilled in the art that the insulin management system 10 may further include other general-purpose components, in addition to the components illustrated in FIG. 1.

The user terminal 1000 refers to a communication terminal capable of using a web service in a wired/wireless communication environment. For example, the user terminal 1000 may be a smartphone, a tablet PC, a PC, a smart TV, a mobile phone, a personal digital assistant (PDA), a laptop, a media player, a micro server, a global positioning system (GPS) device, an e-book reader, a terminal for digital broadcasting, a navigation device, a kiosk, an MP3 player, a digital camera, a home appliance, a device loaded with a camera, and other mobile or non-mobile computing apparatuses.

Also, the user terminal 1000 may be a wearable device having a communication function and a data processing function, such as a watch, glasses, a hair band, and a ring. However, as described above, any terminal loaded with an application capable of internet communication may be adopted without limitation.

The user terminal 1000 may be connected, on a one-to-one basis, to the controller 2000 that is pre-registered. Also, the user terminal 1000 may receive data from the controller 2000 to prevent control from an external device. The user terminal 1000 may transmit, to the controller 2000, configuration information, e.g., system time information, within a preset range.

The controller 2000 may perform a function of transmitting and receiving data to and from the drug injection device 3000, transmit, to the drug injection device 3000, a control signal related to injection of a drug such as insulin, and receive, from the drug injection device 3000, a control signal related to measurement of a biometric value such as glucose.

The controller 2000 may transmit, to the drug injection device 3000, an instruction request to measure a current state of a user and receive measurement data from the drug injection device 3000 as a response to the instruction request.

The drug injection device 3000 also performs a function of measuring a biometric value of the user, such as a glucose measurement value, blood pressure, or a heart rate, but also performs a function of injecting a drug to be injected into the user, such as insulin, glucagon, anesthetic, painkiller, dopamine, growth hormone, or smoking cessation aid.

The drug injection device 3000 may further include a storage unit that stores a material to be periodically injected into the user, and may be controlled so that an injection amount to be injected is injected from the storage unit according to an injection signal generated by the controller 2000.

Here, the drug injection device 3000 may transmit, to the controller 2000, information such as a measurement value and an injection amount. Alternatively, the drug injection device 3000 may transmit a device state message, a biometric value measurement message, a drug injection message, and the like to the controller 2000. For example, the drug injection device 3000 may transmit, to the controller 2000, a device state message including remaining battery capacity information of the drug injection device 3000, whether or not the drug injection device 3000 boots successfully, whether or not injection is successful, and the like. Messages, which are transmitted to the controller 2000, may be transmitted to the user terminal 1000 through the controller 2000. Alternatively, the controller 2000 may transmit, to the user terminal 1000, improved data obtained by processing the received messages.

The drug injection device 3000 may also be implemented to communicate only with the controller 2000 that is pre-registered. In addition, in terms of hardware, the drug injection device 3000 may be divided into a biometric value measurement device that performs a function of measuring a biometric value of the user such as a glucose measurement value, blood pressure, or a heart rate, and an injection device that performs a function of injecting a drug such as insulin, glucagon, or anesthetic. In other words, the biometric value measurement device and the injection device may be present independently. The controller 2000 may be connected to each of the injection device and the biometric value measurement device to generate and provide a control signal for the injection device on the basis of a measurement value measured via the biometric value measurement device.

Meanwhile, the user terminal 1000, the controller 2000, and the drug injection device 3000 may perform communication by using a network. For example, the network may include a local area network (LAN), a wide area network (WAN), a value added network (VAN), a mobile radio communication network, a satellite communication network, and mutual combinations thereof, may be a data communication network in a comprehensive sense, which enables respective network components to smoothly communicate with each other, and may include wired Internet, wireless Internet, and a mobile wireless communication network. In addition, wireless communication may include, for example, wireless LAN (Wi-Fi), Bluetooth, Bluetooth low energy, Zigbee, Wi-Fi Direct (WFD), ultra wideband (UWB), infrared communication (infrared Data Association (IrDA)), near field communication (NFC), and the like, but is not limited thereto.

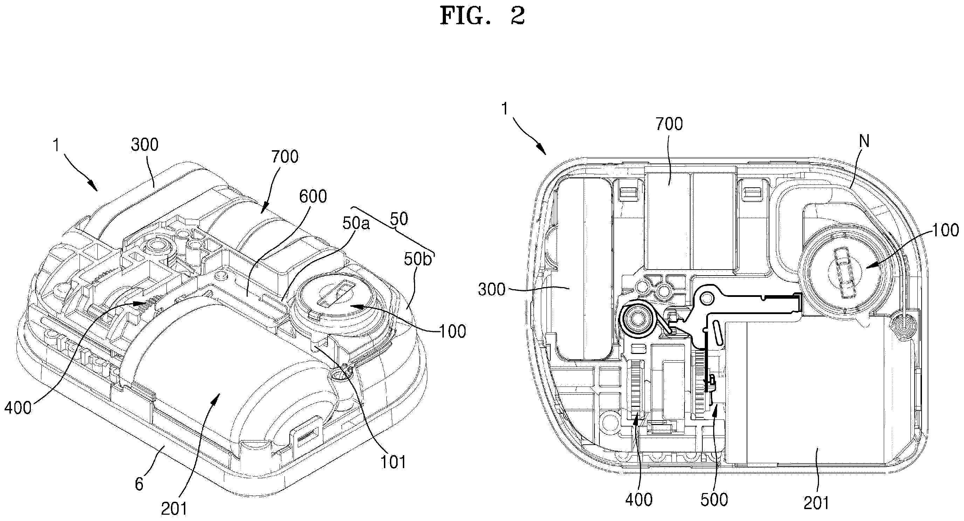

FIG. 2 is a view illustrating an internal structure of a drug injection device according to an embodiment.

Referring to FIG. 2, a drug injection device 1 may include a housing covering the outside and an attachment portion 6 attached to the skin of a user. The drug injection device 1 includes a plurality of components arranged in an inner space between the housing 5 and the attachment portion 6.

The drug injection device 1 may include a base body 50, a needle assembly 100, a storage unit 201, a driving portion 300, a driving unit 400, a clutch unit 500, a trigger member 600, and a battery 700.

The base body 50 forms a basic frame of the housing and is mounted in an inner space of the housing. A plurality of base bodies 50 may be provided. In an embodiment, a first body 50a covering upper portions of internal components and a second body 50b covering lower portions of the internal components may be provided. The first body 50a and the second body 50b may be assembled to fix the internal components of the drug injection device 1 to preset positions. In an embodiment, the base body 50 may be formed as a single integral frame.

The storage unit 201 is mounted on the base body 50 and fluidly connected to the needle assembly 100. Inside the storage unit 201, a plunger (not shown) may linearly move to discharge a drug through a needle N.

The driving portion 300 may generate a driving force and transmit the driving force to the driving unit 400. The driving force transmitted by the driving unit 400 may linearly move the plunger inside the storage unit 201 to discharge the drug.

All types of pumps having a drug suction force and a drug discharge force by electricity may be used as the driving portion 300. For example, all types of pumps, such as a mechanical displacement type micropump and an electromagnetic motion type micropump, may be used. The mechanical displacement type micropump is a pump that uses the movement of solid or fluid, such as gears or diaphragm, to create a pressure difference to induce fluid flow, and includes a diaphragm displacement pump, a fluid displacement pump, a rotary pump, and the like. The electromagnetic motion type micropump is a pump that uses energy in the form of electricity or magnetism directly to move fluid, and includes an electro hydrodynamic pump (EHD), an electro osmotic pump, a magneto hydrodynamic pump, an electro wetting pump, and the like.

When the driving unit 400 is engaged by the clutch unit 500, the driving portion 300 may rotate a driving wheel of the driving unit 400, and the rotation of the driving wheel may allow a rod to linearly move so that the plunger (not shown) may move inside the storage unit 201.

The driving portion 300 may include a membrane arranged inside a cover. The membrane may partition an inner space of the driving portion 300 into a first space and a second space. The driving portion 300 may linearly move a driving shaft by changes in volumes of the first space and the second space.

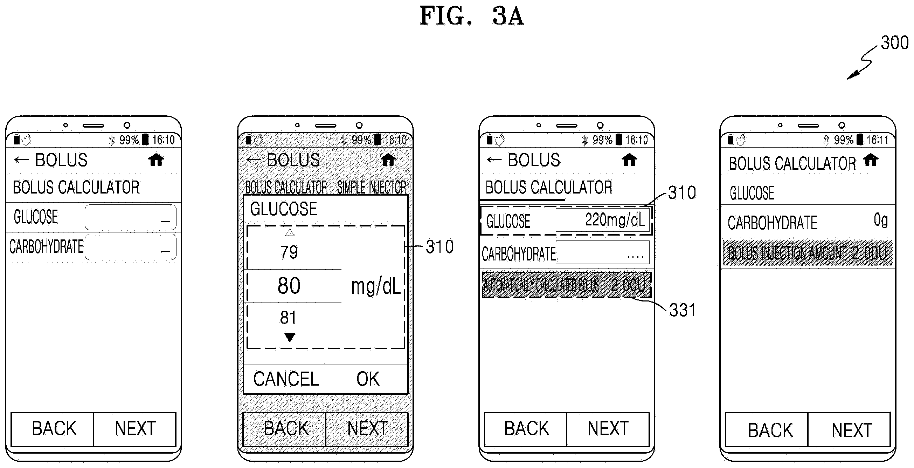

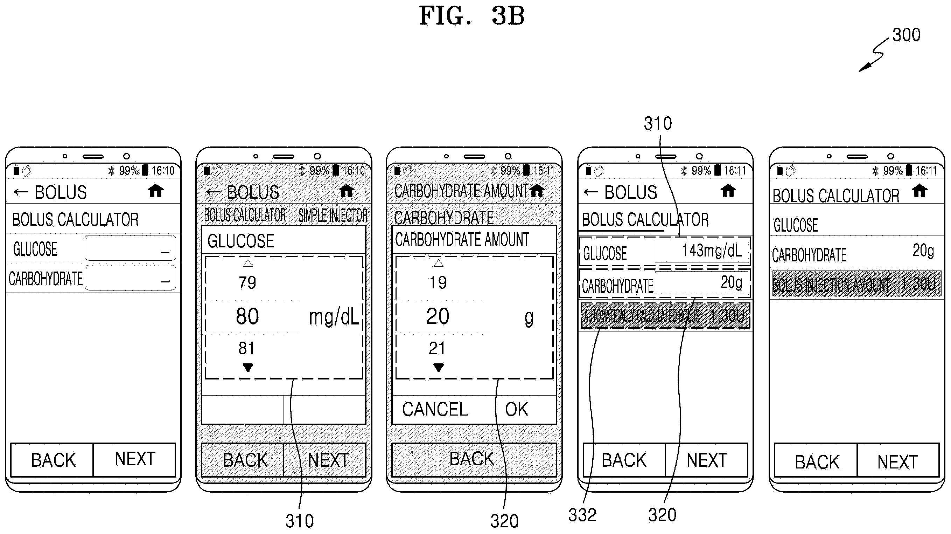

FIGS. 3A and 3B are example views illustrating a method of setting up a drug injection amount calculator, according to an embodiment.

A drug injection amount calculator may be installed in the user terminal 1000, the controller 2000, or the drug injection device 3000 of FIG. 1. The drug injection amount calculator may be a calculator that calculates an insulin amount to be injected into a user separately from basic injection. For example, the drug injection amount calculator may calculate an insulin amount needed to lower glucose that rises due to eating of food or snacks. In addition, the drug injection amount calculator may calculate an insulin amount needed to lower high glucose into a normal glucose range.

As described above, separately from the basic injection of insulin, insulin injected when food intake or high glucose needs to be lowered may be referred to as a bolus. The drug injection amount calculator may be referred to as a bolus calculator, and the bolus calculator may calculate a bolus injection amount.

The bolus injection amount may be determined by various values. For example, the bolus injection amount may be determined by a current glucose level, a carbohydrate-to-insulin ratio, a correction factor, a target glucose level, an insulin amount remaining with an activation time in the body from among a previous bolus injection amount (insulin on board (IOB), bolus on board (BOB) or active insulin), a correction threshold value, an amount of activity, a type and amount of food eaten, and the like.

The correction factor is a value representing a glucose level that may be lowered by 1 unit of bolus insulin. A range of the correction factor is 1 mg/dl/U to 400 mg/dl/U, and may be adjusted in the unit of 1 mg/dl/U. The correction threshold value may refer to the highest glucose level at which an injection of insulin is determined to be needed to control glucose.

The bolus calculator may calculate a bolus injection amount on the basis of an individual bolus profile set value, a current glucose measurement value, an amount of carbohydrates eaten, a residual amount of insulin in the body (IOB), and the like of the user.

In detail, the bolus profile set value may be determined by a target glucose level, a carbohydrate-to-insulin ratio, a correction factor, and an insulin duration. The current glucose measurement value refers to a glucose value measured within 10 minutes.

Referring to FIG. 3A, a user may input a current glucose measurement value 310 into a drug injection amount calculator. The user may omit an input of an amount of carbohydrates eaten. The user may input the current glucose measurement value 310 in units of mg/dl. For example, the user may input 220 mg/dl as the current glucose measurement value 310. The drug injection amount calculator may calculate 2.00 U as a bolus injection amount 331 on the basis of the current glucose measurement value 310. A unit, which may be input as the current glucose measurement value 310, may also be mmol/l, but is not limited to the above unit example.

Referring to FIG. 3B, a user may input a current glucose measurement value 310 and an eaten carbohydrate amount 320 into a drug injection amount calculator. The user may input the current glucose measurement value 310 in units of mg/dl, and input the eaten carbohydrate amount 320 in units of g. For example, the user may input 220 mg/dl as the current glucose measurement value 310 and input 20 g as the eaten carbohydrate amount 320. The drug injection amount calculator may calculate 1.30 U as a bolus injection amount 332 on the basis of the current glucose measurement value 310 and the eaten carbohydrate amount 320.

Meanwhile, the user needs to control glucose that rapidly increases after eating by calculating an optimal insulin injection amount needed to lower glucose that rises due to the food eaten. The user needs to accurately calculate an amount of carbohydrates included in the food eaten to calculate the optimal insulin injection amount. In addition, an optimal insulin injection time point needs to be calculated to appropriately control glucose that increases after a meal.

However, even for the same type of food, calculating an exact amount of carbohydrates may not be easy due to various reasons such as characteristics of a restaurant selling the corresponding food. Also, carbohydrates included in food may have a great effect on a rapid increase in glucose due to eating of food, but an effect of protein or fat included in the food may not be ignored. In addition, different points in time at which carbohydrate, protein, and fat increase glucose of the user need to be considered to calculate the optimal insulin injection amount.

Therefore, when considering the above aspects, an insulin injection amount or an insulin injection time point, which is calculated by the user roughly inputting an amount of carbohydrates according to the type of food, may not be accurate. For example, when more insulin than a needed amount of insulin is injected, a hypoglycemic shock may occur. Alternatively, glucose, which increases at intervals of time after eating food, may not be easily controlled due to protein or fat.

Accordingly, there is a need for a method capable of calculating an optimal insulin injection amount or insulin injection time point by receiving information about food eaten by a user.

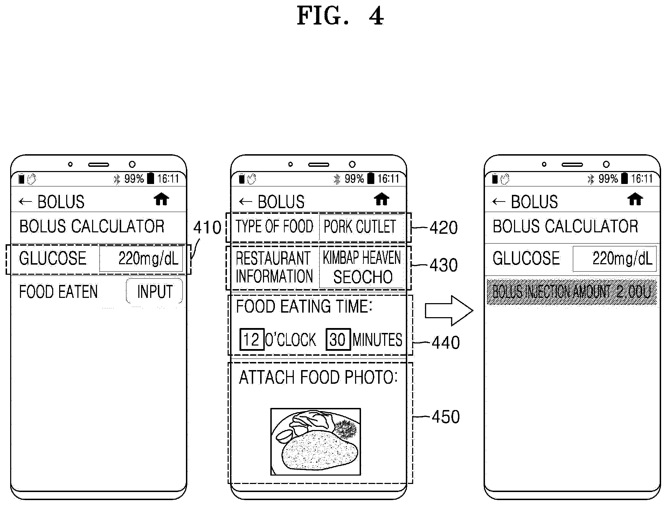

FIG. 4 is an example view illustrating calculating an insulin injection amount on the basis of information about food, according to an embodiment.

According to an embodiment, a drug injection amount calculator included in the user terminal 1000, the controller 2000, or the drug injection device 3000 may receive a current glucose measurement value for a first user from a biometric value measurement device. In addition, the drug injection amount calculator may receive, from the first user, information about food eaten. The drug injection amount calculator may calculate an insulin injection amount on the basis of a glucose measurement value for the first user and the information about food.

Referring to FIG. 4, a drug injection amount calculator may receive a current glucose measurement value for a first user from a biometric value measurement device. For example, the drug injection amount calculator may receive 220 mg/dl as a current glucose measurement value 410. Meanwhile, the drug injection amount calculator may not receive a glucose measurement value from the biometric value measurement device, but the first user may directly input a glucose measurement value via an interface of the user terminal 1000, and information about the input glucose measurement value may be transmitted to the drug injection amount calculator.

In addition, the drug injection amount calculator may receive, from the first user, information about food eaten. For example, the first user may input, via the interface of the user terminal 1000, the information about food eaten, and the input information about food may be transmitted to the drug injection amount calculator.

According to an embodiment, the information about food may include at least one of a type of food eaten by the first user, information about a restaurant selling the food, and information about a point in time of eating the food. Alternatively, the information about food may also include a photo of the food eaten by the first user. Referring to FIG. 4, the drug injection amount calculator may specifically specify the food eaten by the first user by receiving a type 420 of food eaten by the first user, information 430 about a restaurant selling the food, information 440 about a point in time of eating the food, and a photo 450 of the food, and the like.

According to an embodiment, the drug injection amount calculator may determine, from the received information about food, whether or not a specific food is the same as food eaten by the first user in the past. For example, when food eaten by the first user is determined to be the same as food eaten by the first user in the past, the drug injection amount calculator may calculate an insulin injection amount on the basis of at least one of an insulin injection amount injected into the first user after the first user eats the food in the past and information about a change in glucose measurement value for the first user after the first user eats the food in the past.

In other words, the drug injection amount calculator may specifically specify food eaten by the first user from the type of food eaten by the first user, the information about the restaurant selling the food, the photo of the food, and the like. When a specified food is determined to be the same as food eaten by the first user in the past, the drug injection amount calculator may calculate an optimal insulin injection amount by referring to at least one of information about a point in time of eating the food, an insulin injection amount injected into the first user after the first user eats the corresponding food in the past, and information about a change in glucose measurement value for the first user after the first user eats the corresponding food in the past.

Meanwhile, the drug injection amount calculator may also calculate an appropriate insulin injection time point on the basis of the glucose measurement value for the first user and the information about food. For example, when, from the information about food, the specified food is determined to be the same as the food eaten by the first user in the past, the drug injection amount calculator may calculate an appropriate insulin injection time point by referring to at least one of information about a point in time of eating the food, an insulin injection amount injected into the first user after the user eats the corresponding food in the past, and information about a change in glucose measurement value for the first user after the first user eats the corresponding food in the past. Alternatively, the drug injection amount calculator may also calculate a plurality of appropriate insulin injection time points so that a calculated insulin injection amount may be divided and injected at each of the plurality of insulin injection time points, rather than injecting the calculated insulin injection amount at once before eating food. Meanwhile, even when the food eaten by the first user is not the same as the food eaten by the first user in the past and corresponds to food eaten for the first time, an optimal insulin injection amount may also be calculated by using information related to a second user other than the first user.

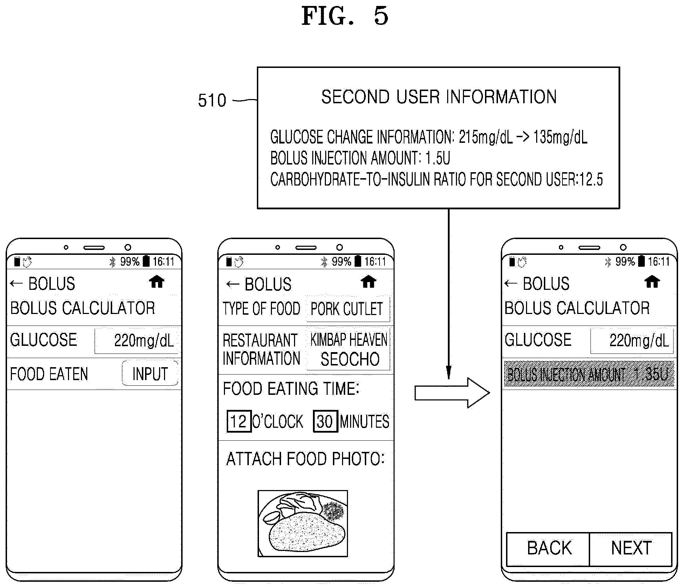

FIG. 5 is an example view illustrating calculating an insulin injection amount on the basis of information about food and information about a second user, according to an embodiment.

According to an embodiment, when food eaten by a first user is determined to not to be the same as food eaten by the first user in the past, a drug injection amount calculator may calculate an insulin injection amount on the basis of at least one of an insulin injection amount injected into a second user after the second user eats the corresponding food in the past, information about a change in glucose measurement value for the second user after the second user eats the corresponding food in the past, and a carbohydrate-to-insulin ratio set for each user.

In other words, the drug injection amount calculator may specifically specify food eaten by the first user from a type of food eaten by the first user, information about a restaurant selling the food, a photo of the food, and the like, and, when the specified food is determined not to be the same as food eaten by the first user in the past, may calculate an optimal insulin injection amount by referring to information about a point in time of eating the food, an insulin injection amount injected into the second user after the second user eats the corresponding food in the past, information about a change in glucose measurement value for the second user after the second user eats the corresponding food in the past, and a carbohydrate-to-insulin ratio set for each user. The carbohydrate-to-insulin ratio set for each user may include a carbohydrate-to-insulin ratio for the first user and a carbohydrate-to-insulin ratio for the second user.

For example, referring to FIG. 5, second user information 510 may include information indicating that a glucose measurement value for the second user changes from 215 mg/dL to 135 mg/dL by injecting insulin of 1.5 U after the second user eats the same food as food eaten by the first user in the past, and a carbohydrate-to-insulin ratio for the second user is 12.5. The drug injection amount calculator may calculate an optimal insulin injection amount by considering the second user information 510 and a carbohydrate-to-insulin ratio for the first user.

Meanwhile, the drug injection amount calculator may also calculate an appropriate insulin injection time point on the basis of a glucose measurement value for the first user, information about food, and the second user information 510. For example, when, from the information about food, the specified food is determined not to be the same as the food eaten by the first user in the past, the drug injection amount calculator may calculate the appropriate insulin injection time point by referring to at least one of information about a point in time of eating the food, an insulin injection amount injected into the second user after the second user eats the corresponding food in the past, information about a change in glucose measurement value for the second user after the second user eats the corresponding food in the past, and a carbohydrate-to-insulin ratio set for each user. Alternatively, the drug injection amount calculator may also calculate a plurality of appropriate insulin injection time points so that a calculated insulin injection amount may be divided and injected at each of the plurality of insulin injection time points, rather than injecting the calculated insulin injection amount at once before eating food.

Meanwhile, a glucose measurement value acquired from a glucose measurement device may have an error range according to an error of the glucose measurement device or physical characteristics of a user. Therefore, a more accurate insulin injection amount needs to be calculated by considering the error range.

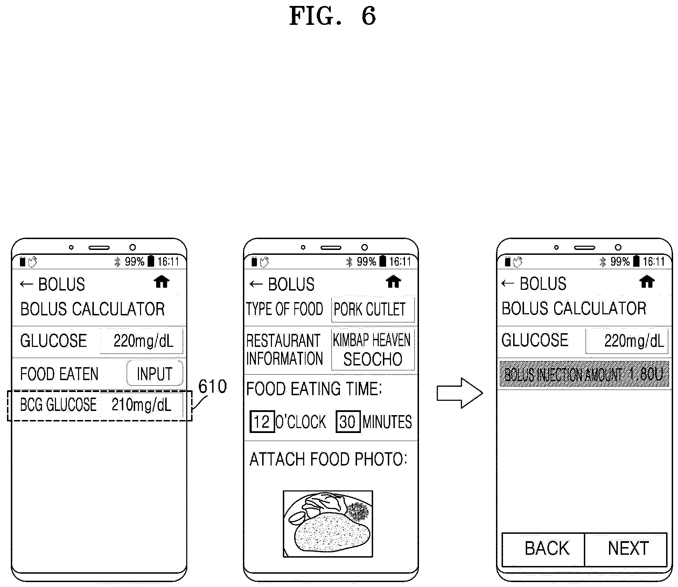

FIG. 6 is an example view illustrating calculating an insulin injection amount on the basis of an error range, according to an embodiment.

According to an embodiment, a drug injection amount calculator may calculate an error range on the basis of a difference value between a glucose measurement value received from a biometric value measurement device and a glucose measurement value measured by a blood glucose monitoring (BGM) method. Also, the drug injection amount calculator may calculate an insulin injection amount on the basis of the error range.

For example, referring to FIG. 6, the drug injection amount calculator may receive a current glucose measurement value for a first user from the biometric value measurement device. For example, the drug injection amount calculator may receive 220 mg/dl as the current glucose measurement value. Here, the biometric value measurement device may correspond to a device for measuring glucose of a user by a continuous glucose monitoring (CGM) method. The continuous glucose monitoring method is a method in which a sensor is attached to the subcutaneous fat of the abdomen and the glucose in the cell interstitial fluid is measured via the sensor.

Also, the drug injection amount calculator may receive, from the first user, a glucose measurement value measured by the BGM method. For example, the first user may directly input, via an interface of the user terminal 1000, the glucose measurement value measured by the BGM method, and information about the glucose measurement value measured by the BGM method may be transmitted to the drug injection amount calculator.

The drug injection amount calculator may calculate an error range of a glucose measurement value received from the biometric value measurement device, on the basis of a difference between the glucose measurement value received from the biometric value measurement device for measuring glucose by a continuous glucose monitoring method and a glucose measurement value measured by a blood glucose monitoring (BGM) method. The drug injection amount calculator may calculate an insulin injection amount on the basis of the error range. In other words, the insulin injection amount calculated as described above with reference to FIGS. 3A to 5 may be corrected by considering the error range.

Similarly, the drug injection amount calculator may calculate an insulin injection time point on the basis of the error range, and the insulin injection time point calculated as described above with reference to FIGS. 3A to 5 may be corrected by considering the error range.

Referring to FIG. 6, the drug injection amount calculator may receive information indicating that a current glucose measurement value for the first user corresponds to 220 mg/dL, from the biometric value measurement device for measuring glucose by the continuous glucose monitoring method. Also, the drug injection amount calculator may receive, from the first user, information indicating that a glucose measurement value 610 measured by a BGM method corresponds to 210 mg/dL. The drug injection amount calculator may calculate an optimal insulin injection amount on the basis of information indicating that a difference between a glucose measurement value received from the biometric value measurement device for measuring glucose by the continuous glucose monitoring method and the glucose measurement value 610 measured by the BGM method is 10 mg/dL, and information about food, which is received from the first user.

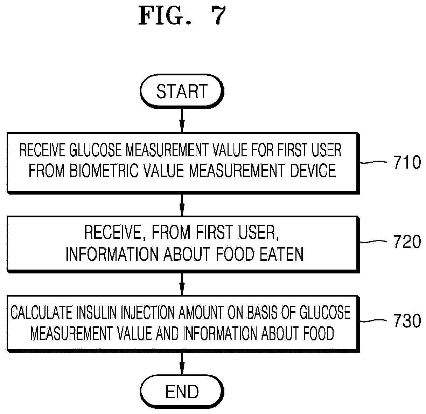

FIG. 7 is a flowchart of a method of calculating an insulin injection amount, according to an embodiment.

A controller may communicate with a drug injection device. The controller may be a component included in a user terminal such as a smartphone or a PC, or may be a component independent of the user terminal. Alternatively, the controller may be a component included in the drug injection device. Hereinafter, the drug injection device and the controller may perform communication by using a network.

In operation 710, the controller may receive a glucose measurement value for a first user from a biometric value measurement device. For example, the biometric value measurement device may correspond to a device for measuring glucose of a user by a continuous glucose monitoring (CGM) method. The continuous glucose monitoring method is a method in which a sensor is attached to the subcutaneous fat of the abdomen and the glucose in the cell interstitial fluid is measured via the sensor.

In operation 720, the controller may receive, from the first user, information about food eaten. According to an embodiment, the information about food may include at least one of information about a type of food, information about a restaurant selling the food, and information about a point in time of eating the food.

In operation 730, the controller may calculate an insulin injection amount on the basis of the glucose measurement value and the information about food.

According to an embodiment, the controller may determine whether the food is the same as food eaten by the first user in the past, and may calculate the insulin injection amount on the basis of the determination. When the food is determined to be the same as the food eaten by the first user in the past, the controller may calculate the insulin injection amount on the basis of at least one of an insulin injection amount injected into the first user after the first user eats the food in the past and information about a change in glucose measurement value for the first user after the first user eats the food in the past.

According to an embodiment, the controller may determine whether or not the food is the same as the food eaten by the first user in the past, and may calculate the insulin injection amount on the basis of the determination. When the food is determined not to be the same as the food eaten by the first user in the past, the controller may calculate the insulin injection amount on the basis of an insulin injection amount injected into a second user after the second user eats the food in the past, information about a change in glucose measurement value for the second user after the second user eats the food in the past, and a carbohydrate-to-insulin ratio set for each user.

According to an embodiment, the controller may calculate an error range on the basis of a difference value between a glucose measurement value received from a glucose measurement device and a glucose measurement value measured by a blood glucose monitoring (BGM) method, and may calculate the insulin injection amount on the basis of the error range.

Various embodiments of the present disclosure may be implemented as software (e.g., a program) including one or more instructions stored in a storage medium readable by a machine. For example, a processor of the machine may call, from the storage medium, at least one instruction from among the stored one or more instructions and execute the same. This enables the machine to be operated to perform at least one function according to the called at least one instruction. The one or more instructions may include code generated by a compiler or code executable by an interpreter. The machine-readable storage medium may be provided in the form of a non-transitory storage medium. Here, "non-transitory" only means that a storage medium is a tangible device and does not include signals (e.g., electromagnetic waves), and this term does not distinguish between a case in which data is semi-permanently stored in the storage medium and a case in which data is temporarily stored.

According to an embodiment, the method according to various embodiments of the present disclosure may be included and provided in a computer program product. The computer program product may be traded between a seller and a buyer as a product. The computer program product may be distributed in the form of a machine-readable storage medium (e.g., compact disc read only memory (CD-ROM)), or may be online distributed (e.g., downloaded or uploaded) via an application store (e.g., Play Store^{™}) or directly between two user devices. In the case of online distribution, at least a portion of the computer program product may be at least temporarily stored or temporarily generated in a machine-readable storage medium such as a server of a manufacturer, a server of an application store, or a memory of a relay server.

In addition, as used herein, "unit" may be a hardware component such as a processor or a circuit, and/or a software component executed by a hardware component such as a processor.

The scope of the present embodiment should be defined by claims described below rather than the detailed description, and should be construed as including all changes or modifications derived from the meaning and scope of the claims and an equivalent concept thereof.

## Claims

1. A method of calculating an insulin injection amount, the method comprising:
receiving a glucose measurement value for a first user from a biometric value measurement device;
receiving, from the first user, information about food eaten; and
calculating an insulin injection amount on the basis of the glucose measurement value and the information about food.

2. The method of claim 1, wherein the information about food includes at least one of information about a type of food, information about a restaurant selling the food, and information about a point in time of eating the food.

3. The method of claim 1, wherein the calculating includes:
determining whether or not the food is the same as food eaten by the first user in the past; and
calculating the insulin injection amount on the basis of the determination, wherein, when the food is determined to be the same as the food eaten by the first user in the past, the insulin injection amount is calculated on the basis of an insulin injection amount injected into the first user after the first user eats the food in the past and information about a change in glucose measurement value for the first user after the first user eats the food in the past.

4. The method of claim 1, wherein the calculating includes:
determining whether or not the food is the same as food eaten by the first user in the past; and
calculating the insulin injection amount on the basis of the determination, wherein, when the food is determined not to be the same as the food eaten by the first user in the past, the insulin injection amount is calculated on the basis of an insulin injection amount injected into a second user after the second user eats the food in the past, information about a change in glucose measurement value for the second user after the second user eats the food in the past, and a carbohydrate-to-insulin ratio set for each user.

5. The method of claim 1, wherein the calculating includes:
calculating an error range on the basis of a difference value between a glucose measurement value received from the biometric value measurement device and a glucose measurement value measured by a blood glucose monitoring (BGM) method; and
calculating an insulin injection amount on the basis of the error range.

6. A device for calculating an insulin injection amount, the device comprising:
a memory configured to store at least one program; and
a processor configured to perform a calculation by executing the at least one program, wherein the processor is configured to receive a glucose measurement value for a first user from a biometric value measurement device, receive, from the first user, information about food eaten, and calculate an insulin injection amount on the basis of the glucose measurement value and the information about food.

7. A computer program product comprising one or more computer-readable recording media configured to store a program for performing:
receiving a glucose measurement value for a first user from a biometric value measurement device;
receiving, from the first user, information about food eaten; and
calculating an insulin injection amount on the basis of the glucose measurement value and the information about food.
